# EUROPEAN PATENT APPLICATION

(11) **EP 1 357 490 A1**
(43) Date of publication of application: **29.10.2003**
(21) Application number: 01272303.7
(22) Date of filing: 25.12.2001
(51) Int. Cl.: G06F 17/60

(54) **EXAMINATION MANAGEMENT DEVICE**

(30) Priority: 22.12.2000 JP 2000404532
(71) Applicant: ARKRAY, Inc., Kyoto-shi, Kyoto 601-8045 (JP)
(72) Inventor: HYOUDOU, Hiroshi, Kyoto-shi, Kyoto 601-8045 (JP); WADA, Atsushi, Kyoto-shi, Kyoto 601-8045 (JP)
(74) Representative: Piésold, Alexander J.
(86) International application number: JP0111321
(87) International publication number: WO02052468

(57) **Abstract**

When a medical institution outsources an examination to an external examination institution, a transport identification number generated by a number generating section (16) of the medical institution's data management system (1) is printed out on a barcode label and affixed to a specimen container. The transport identification number is sent along with examination instruction information to a database server (3), and based on the transport identification number read from the barcode label affixed to the specimen container sent by the medical institution, a data management system (2) of the external examination institution obtains examination instruction information from the database server (3) and examinations are performed. The examination results are stored on the database server (3) along with the transport identification number. Based on the transport identification number, the data management system (1) of the medical institution obtains the examination results from the database server (3).

## Description

### TECHNICAL FIELD

The present invention relates to examination management devices and examination management systems for managing, for example, the examination instructions and examination data of clinical examinations in medical institutions and the like.

### BACKGROUND ART

Clinical examinations for measuring the constituent concentrations within body fluids such as blood and urine are performed in medical institutions such as hospitals and clinics in order to determine the health conditions of patients. From the perspective of the efficiency and cost of the clinical examination, tests requiring urgency are increasingly handled within the medical institution, and other tests are outsourced to clinical examination centers and other such external examination institutions.

Furthermore, due to the advances and increasingly low cost of computers in recent years, there is a continuing widespread adoption in hospitals of data management systems in which patient data is managed on computers within the hospital. In these data management systems, each individual patient is issued an identification number and records of the patient's medical records and examination data are kept. For example, management of the results for a new examination can be made easy by inputting via the key input section of the measurement device the identification number of a patient who is registered in the data management system, so that the measurement device can print out the results with the identification number. Or the patient's identification number can be printed on a barcode label, which can be affixed to the specimen container. Before taking measurements, the measurement device reads the barcode label with a barcode reader, and then prints out the results along with the patient identification number recorded on the barcode. The results are then input to the data management system with the patient identification numbers as a handle while viewing the printed results.

In even more advanced systems, the data management system and the measurement device are connected by a communications line. Before measuring the specimen, the measurement device receives the patient identification number from the data management system. Once the measurements have been taken, the data is sent to the data management system along with the patient identification number.

When examinations are performed by clinical examination centers and other such external examination institutions, an identification number issued by the external examination institution is affixed as a barcode label to the specimen container, and the request for examination is made in a form to which the same identification number is affixed, and on which is recorded the patient's name, the patient identification number used by the medical institution, as well as the requested examination items and so on. The results are returned to the medical institution by fax or similar methods, and are then managed by being input into the medical institution's data management system.

In this way it can be seen that, due to differences between the medical institution's data management system and the system(s) of the external examination institution(s), achieving centralized data management is very difficult. Even granted that centralized data management is technologically achievable, security issues remain due to the risk of leaking confidential information from the medical institution, such as the patient's name and medical records.

### DISCLOSURE 0F INVENTION

The present invention was conceived to solve the problems described above, and it is an object thereof to provide an examination management device and an examination management system wherein there is no necessity for confidential information such as the individual names of patients or clinical results to leave the medical institution, and that can facilitate the data management for examination results outsourced to external examination institutions.

In order to achieve this object, the examination management device of the present invention is provided with a patient data storage section that stores personal medical data for each patient in association with a unique individual identifier given to each patient, a control section that performs different processing in response to whether an examination should be conducted within the medical institution or whether it should be outsourced to an external examination institution, an identifier issuing section that issues a transport identifier, and an identifier storage section that stores the issued transport identifier in association with the individual identifier. The control section outputs an examination instruction, including the individual identifier, for the use of the examination device inside the medical institution when the examination is one that should be conducted within the medical institution, and when the examination is one that should be outsourced to an external examination institution, it causes the identifier issuing section to issue a transport identifier to be appended to a specimen, causes the identifier storage section to store the issued transport identifier in association with the individual identifier of the patient from whom the specimen was collected, and outputs an examination instruction for an external examination institution.

In the above-described configuration, since a transport identifier is used for the exchange of specimens and data between the medical institution using this examination management device and the external examination institution, even when the medical institution is using a data management system that is unique to the examination institution, and when the external examination institution is using a data management system that is unique to the examination institution, the data of both parties can be queried easily with the transport identifier. Furthermore, confidential information such as the individual names of patients is not let out from the hospital, so even supposing that examination results were to be leaked, since it cannot be specified whose examination results they are, the confidentiality of patient data can be protected.

Furthermore, in order to achieve the above described object, the examination management system of the present invention includes the examination management device of the present invention described above, as well as an examination data storage device that can be accessed by both the examination management device and the examination device of the external examination institution. In this examination management system, the examination management device causes the examination instruction including the transport identifier to be sent to and stored at the examination data storage device; the examination device of the external examination institution obtains from the examination data storage device the examination instruction information corresponding the transport identifier appended to the specimen, performs an examination based on the obtained examination instruction information, and causes the result to be stored to the examination information storage device in association with the transport identifier; and the examination management device obtains from the examination information storage device the examination result based on the transport identifier.

With this configuration, since a transport identifier is used for the exchange of specimens and data between the medical institution using this examination management device and the external examination institution, even when the medical institution is using a data management system that is unique to the examination institution, and when the external examination institution is using a data management system that is unique to the examination institution, the data of both parties can be queried easily with the transport identifier. Furthermore, confidential information such as the individual names of patients is not let out from the hospital, so even supposing that examination results were to become leaked, since it cannot be specified whose examination results they are, the confidentiality of patient data can be protected. Moreover, as examination instruction information including a transport identifier is exchanged between the medical institution and the external examination institution via an examination data storage device, there is also the advantage of very reliable prevention against the loss of confidential patient data to the external examination institution.

### BRIEF DESCRIPTION OF DRAWING

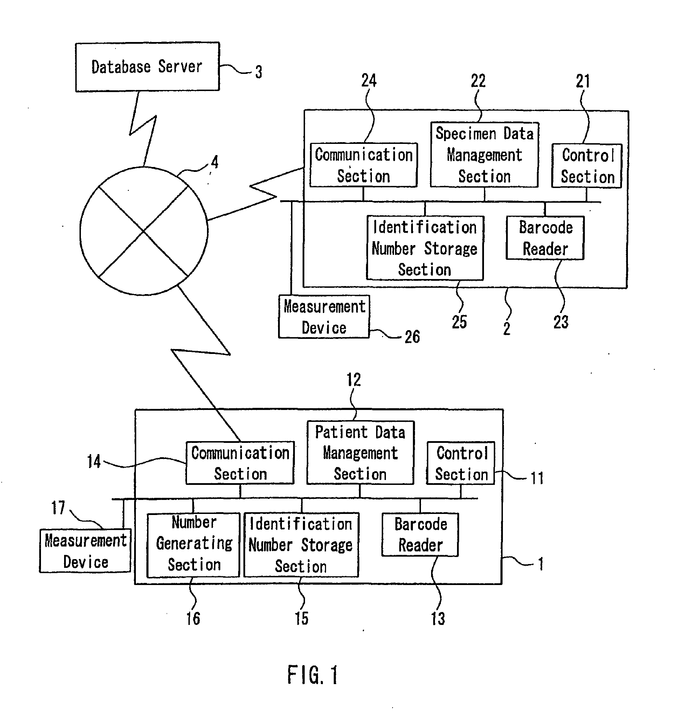

FIG. 1 is a block diagram that shows the configuration of a system according to the present invention.

### BEST MODE F0R CARRYING OUT THE INVENTION

Referring to the accompanying drawing, the following is an explanation of embodiments of the present invention.

In FIG. 1, the reference number 1 refers to a medical institution's internal data management system, 2 refers to an external institution's data management system that is set up in an examination institution outside the medical institution, and 3 refers to a database server. The medical institution data management system 1, the external institution's data management system 2, and the database server 3 are connected by a network 4 such as the Internet.

The medical institution data management system 1 is provided with a control section 11 that performs control for the entire system, a patient data management section 12 that manages patient data, a barcode reader 13 that reads a barcode label on which is provided an identification number for transport or the like, a communication section 14 that enables external communication, a number generating section 16 that generates an identification number for transport, and an identification number storage section 15 that associates and stores the transport identification number with a patient identification number stored in the patient data management section 12.

The communication section 14 is connected to the external database server 3 via a firewall (not shown in diagram). Personal information of the patient, such as name and date of birth, past medical records, and other information such as examination data and so on are stored in the patient data management section 12 in association with a patient identification number that is given to each individual patient. Furthermore, the patient data management section 12 is connected to a measurement device 17 installed in the medical institution, with which it can exchange data such as measurement data and patient identification numbers. As will be explained later, in the event of outsourcing an examination to an external examination institution, the number generating section 16 generates a transport identification number that is attached to the specimen for transport to the examination institution.

The external institution's data management system 2 is provided with a control section 21 that performs control for the entire system, a specimen data management section 22 that manages the measurement data of specimens, a barcode reader 23 that reads a barcode label on which is provided an identification number for transport or the like, a communication section 24 that enables external communication, and an identification number storage section 25 that associates and stores a transport identification number with a specimen identification number stored in the specimen data management section 22.

The communication section 24 is connected to the external database server 3 via a firewall (not shown in diagram). Specimen measurement data and specimen identification numbers are associated and stored in the specimen data management section 22. Furthermore, the specimen data management section 22 is connected to a measurement device 26 installed in the external examination institution, with which it can exchange data such as specimen measurement data and specimen identification numbers.

The database server 3 associates and stores transport identification numbers, measurement item information, and specimen measurement data. In the present embodiment, the database server 3 is a separate system to the data management system 2 of the external examination institution, but it also may be set up within the external examination institution's data management system 2.

The following is an explanation of an examination procedure. Blood is collected from a patient at a medical institution in order to carry out blood tests. Examination items that call for urgency are handled by the measurement device 17 inside the medical institution, and examinations for items other than those are performed by an external examination institution, so the collected blood is poured into two specimen containers. The orders for the examination items are stored in the patient data management section 12 after appending to them the indication of whether the item is to be performed inside the medical institution or whether the item is to be outsourced to an external examination institution.

For the measurements to be taken inside the medical institution, the measurement orders are sent with the patient identification number to the measurement device 17 by the patient data management section 12 of the data management system 1 inside the medical institution. After receiving the measurement instructions, and when the specimen container is in place, the measurement device 17 retrieves the specimen from the specimen container and performs measurements. The results of the measurements are associated with the patient identification number and sent to the patient data management section 12 of the data management system 1 inside the medical institution. The patient data management section 12 stores the sent measurement data.

On the other hand, in the case of items whose examination is outsourced to an external examination institution, a transport identification number generated by the number generating section 16 is issued as a barcode that is printed out on a barcode label by a printing device (not shown in diagram) of the medical institution's data management system 1. This barcode label is affixed to the specimen container and dispatched to the external examination institution. It should be noted that the transport identification number generated by the number generating section 16 may be a completely randomly assigned number, but it also may be an assigned number that includes some information related to the medical institution or information related to the patient.

Before dispatching the specimen container to the external examination institution, the transport identification number on the barcode label affixed to the specimen container is read by the barcode reader 13 at the medical institution, along with the patient identification number indicated by the barcode on the patient's medical records. The transport identification number and the patient identification number that have been read in are associated and stored in the identification number storage section 15. It should be noted that, although the patient identification number referred to here is a patient identification number indicated by a barcode on the patient's medical records, there is no limitation in this respect. Other conceivable arrangements include, for example in the case of the data management system 1 of a medical institution managing patient medical records within an electronic medical record system, an arrangement in which the patient identification number for a given patient is obtained from the electronic medical record system when the transport identification number on the specimen container is read by the barcode reader 13.

The control section 11 obtains the list of items whose examination is outsourced to an external examination institution from the patient data management section 12, and the code of the facility to which the request is made and the transport identification number are associated and sent from the communication section 14 to the database server 3.

When the specimen container with a barcode label containing the transport identification number is received at the external examination institution, the barcode label is read by the barcode reader 23. The transport identification number that has been read in is registered in the specimen data storage section 22 and given a specimen identification number for management purposes within the examination institution. The control section 21 queries the database server 3 in regard to the transport identification number via the communication section 24, and then obtains from the database server 3 the examination item information that has been stored in association with that transport identification number.

A barcode label with the specimen identification number given for management purposes is issued at the examination institution and affixed to the specimen container, and the specimen container is placed in a transport section of the measurement device 26. The specimen identification number is read from the barcode label affixed to the specimen container by a barcode reader (not shown in diagram) of the measurement device 26.

The measurement device 26 queries the specimen data management section 22 in regard to the specimen identification number and performs measurements for the examination items included in the examination item information obtained from the database server 3. The measurement results are sent together with the specimen identification number to the specimen data management section 22. The specimen data management section 22 stores the sent measurement results. Based on the transport identification number and specimen identification number stored in the identification number storage section 25, as well as the specimen identification number and measurement results stored in the specimen data management section 22, the control section 21 causes the communication section 24 to send to the database server 3 the transport identification number in association with the measurement data.

When the database server 3 receives the transport identification number and the measurement data from the external examination institution, it stores the received transport identification number and measurement data, and also sends these to the data management system 1 of the medical institution.

When the transport identification number and measurement data are received by the communication section 14 at the medical institution's data management system 1, the patient data management section 12 acknowledges the patient identification number for the received transport identification number by referring to the identification number storage section 15. The acknowledged patient identification number and the received measurement data are associated and stored within the patient data management section 12.

It should be noted that when the transport identification number is assigned including some information related to the medical institution or information related to the patient, it is also possible to obtain the patient's past measurement data from the database server 3 by querying the database server 3 for the measurement data, taking the information related to the medical institution or information related to the patient as a handle. In this case, when an individual patient is using multiple medical institutions, it becomes easy for these medical institutions to share measurement data.

It should also be noted that, although the Internet is given in this embodiment as an example of the network 4, there is no limitation to the Internet, and it is possible to use any network. Furthermore, this embodiment illustrated an example in which a transport identifier and an individual identifier are both given as numbers, but both the transport identifier and the individual identifier can be given as numerals, characters, or symbols, or combinations of these.

### INDUSTRIAL APPLICABILITY

As shown above, by associating transport identification numbers with specimens and measurement data and exchanging them between a medical institution and an external examination institution, the present invention can provide a data management device that makes it easy to collate measurement data even when these institutions both use internal identification numbers for management purposes. With this data management device, no information about the individual patient goes outside the medical institution, and it therefore has the effect of allaying concerns about leaking confidential information of individual patients.

## Claims

1. An examination management device comprising:
a patient data storage section that stores personal medical data for each patient in association with a unique individual identifier given to each patient;
a control section that performs different processing in response to whether an examination should be conducted within the medical institution or whether it should be outsourced to an external examination institution;
an identifier issuing section that issues a transport identifier; and
an identifier storage section that stores the issued transport identifier in association with the individual identifier of the patient;
wherein the control section:
outputs an examination instruction, including the individual identifier, for the use of the examination device inside the medical institution when the examination is one that should be conducted within the medical institution; and
when the examination is one that should be outsourced to an external examination institution, causes the identifier issuing section to issue a transport identifier to be appended to a specimen, causes the identifier storage section to store the issued transport identifier in association with the individual identifier of the patient from who the specimen was collected, and outputs an examination instruction for external examination institution.

2. The examination management device according to claim 1,
wherein the examination management device is connected to the examination device inside the medical institution via a communications means,
the examination management device further comprising a communications section that, via the communications means, sends the examination instruction to the examination device, and receives from the examination device the examination result of each patient in association with the transport identifier,
wherein the control section extracts from the identifier storage section the individual identifier corresponding to the transport identifier associated with the received examination result, and causes the patient data storage section to store the examination result in association with the extracted individual identifier.

3. The examination management device according to claim 1,
wherein the identifier issuing section issues, as the transport identifier, an identifier made of random numerals, characters, or symbols, or combinations of these.

4. The examination management device according to claim 1,
wherein the identifier issuing section issues, as the transport identifier, an identifier that includes the individual identifier of the patient from whom the specimen has been collected.

5. An examination management system comprising the examination management device according to any of the claims 1 to 4, and an examination data storage device that can be accessed by both the examination management device and the examination device of the external examination institution,
wherein the examination management device causes the examination instruction including the transport identifier to be sent to and stored at the examination data storage device,
wherein the examination device of the external examination institution obtains from the examination data storage device the examination instruction information corresponding to the transport identifier appended to the specimen, performs an examination based on the obtained examination instruction information, and causes the result to be stored in the examination information storage device in association with the transport identifier, and
wherein the examination management device obtains from the examination information storage device the examination result based on the transport identifier.

6. The examination management system according to claim 5, wherein the examination information storage device is a device outside the examination device.

7. The examination management system according to claim 5, wherein the examination device of the external examination institution includes the examination information storage device as an internal device.

8. An examination management system comprising the examination management device according to claim 4, and an examination data storage device that can be accessed by both the examination management device and the examination device of the external examination institution,
wherein the examination management device causes the examination instruction including the transport identifier to be sent to and stored at the examination information storage device,
wherein the examination device of the external examination institution obtains from the examination data storage device the examination instruction information corresponding to the transport identifier appended to the specimen, performs an examination based on the obtained examination instruction information, and causes the result to be stored in the examination information storage device in association with the transport identifier, and
wherein the examination management device obtains from the examination information storage device the examination result based on the individual identifier of each patient.
